# EUROPÄISCHE PATENTSCHRIFT

(11) **EP 0 697 901 B1**
(45) Veröffentlichungstag und Bekanntmachung des Hinweises auf die Patenterteilung: **26.01.2000**
(21) Anmeldenummer: 93912707.2
(22) Anmeldetag: 14.05.1993
(51) Int. Cl.: A61N 5/06

(54) **SICHERHEITS-LASERSTRAHLAUSTRITTSÖFFNUNGEN (SLA) FÜR MEDIZINISCHE THERAPIE- UND DIAGNOSEGERÄTE**
SAFETY LASER BEAM APERTURES (SLA) FOR THERAPEUTIC AND DIAGNOSTIC MEDICAL EQUIPMENT
OUVERTURES DE SORTIE DE FAISCEAU LASER A DISPOSITIF DE SECURITE POUR APPAREILS MEDICAUX THERAPEUTIQUES ET DIAGNOSTIQUES

(43) Veröffentlichungstag der Anmeldung: 28.02.1996
(73) Patentinhaber: Oppold, Eberhard, Dipl.-Ing., 54293 Trier (DE)
(72) Erfinder: Oppold, Eberhard, Dipl.-Ing., 54293 Trier (DE)
(86) Internationale Anmeldenummer: EP9301190
(87) Internationale Veröffentlichungsnummer: WO9428972

(56) Entgegenhaltungen:
- EP-A- 0 172 490
- EP-A- 0 435 506
- WO-A-91/18646
- DE-A- 2 548 354
- DE-A- 3 719 561
- DE-B- 2 619 930
- DE-U- 8 628 810
- DE-U- 8 701 528
- FR-A- 1 495 668
- FR-A- 2 329 258
- FR-A- 2 589 067
- US-A- 4 672 969
- US-A- 4 854 320
- US-A- 4 973 848
- US-A- 5 002 051
- US-A- 5 140 984
- US-A- 5 150 704
- US-A- 5 200 604

## Beschreibung

### 1.) Technisches Gebiet

Die vorliegende Erfindung bezieht sich auf Sicherheits-Laserstrahlaustrittsöffnungen (SLA) für medizinische Therapie- und Diagnosegeräte im Leistungsbereich der Mid- und Softlaser nach dem Oberbegriff des Anspruch 1.

Die SLA sollen Laser-Therapie- und Diagnosegeräte im medizinischen Bereich ermöglichen, die als eigensichere Systeme entsprechend der national unterschiedlich strengen Laser - Sicherheitsbestimmungen eingesetzt werden dürfen. Es wird der Einsatz außerhalb medizinischer Behandlungsräume und/oder ohne ständige Aufsicht von medizinischem Fachpersonal möglich.

### 2.) Stand der Technik

Es sind Mid- und Softlaser - Geräte für den Einsatz in der medizinischen Praxis bekannt, die Sicherheitseinrichtungen entsprechend nationaler und internationaler Laser - Sicherheitsvorschriften haben entsprechend der zum Beispiel vom Technischen Komitee Nr.76 der IEC ( Internationale Elektrotechnische Kommission) ausgearbeiteten Norm "Laser - Einrichtungen".

Der Text dieser Norm beruht auf den Schriften:
1. 6-Monats-Regel 76 (CO) 6
2. Abstimmungsbericht 76 (CO) 7
3. 2-Monats-Verfahren 76 (CO) 8
4. Abstimmungsbericht 76 (CO) 11.

Die IEC-Norm "**Laser** - Einrichtungen" findet sich zum Teil vollständig wieder in unterschiedlichen nationalen Normen und Vorschriften, zum Beispiel:

### USA

1.American National Standard for the safe use of lasers in health care facilities ANSI Z136.3-1988
2.Performance Standard for Light-Emitting Products, Part 1040, der Food and Drug Administration.

### Deutschland

1.DIN/VDE 0836, VDE-Bestimmung für die elektrische Sicherheit von Lasergeräten und-Anlagen.
2.DIN/VDE 0837, Strahlungssicherheit von Lasereinrichtungen, Klassifizierung von Anlagen, Anforderungen, Benutzer-Richtlinien.

### Europa

Harmonisierungsdokument "Elektrische Sicherheit von Lasergeräten und -Anlagen" für die europäischen Länder, herausgegeben vom Europäischen Normen Komitee CENELEC NK 1.

### England

1.BS 4803 Radiation Safety of Laser Products and Systems, herausgegeben vom British Standards Institution
2.Guidance on the safe use of lasers in medical practice, herausgegeben vom DHSS (Department of Health and Social Security).

### Frankreich

Merkblatt: Les lasers; Risques et moyens de protection (ND 1607-125-86), herausgegeben vom INRS (Institut National de Recherche et de Securite).

Die einzelnen Normen und Regeln legen die MZB (Maximal zulässige Bestrahlung) oder MPE (Maximum Permissible Exposure) fest, die für die Augen und die Haut unterschiedlich hoch sind in Abhängigkeit von der Wellenlänge der Strahlung, der Impulsdauer, der Energiedichte und der gesamten Expositionszeit.

Die Laser sind fast weltweit in vier Gefahrenklassen eingeteilt, wobei Klasse I für eigensichere Systeme bestimmt ist, die die geforderten MZB-Werte keinesfalls überschreiten können. Klasse II bezeichnet die Geräte mit niedriger Leistung (cw ≦ 1 mW) und Laserwellenlängen im sichtbaren Bereich.

Saft- und Mid-Laser entsprechend der Int. Pat.Klasse A 61 N 5/06 sind fast alle der Laser-Klasse IIIB zuzuordnen, die einen Strahlungsgrenzwert von 500 mW bei cw-Betrieb zuläßt bei zugleich unsichtbarer wie auch sichtbarer Laserstrahlung. Eine Ausnahme stellt der in EP 0416 150 A1 vorgestellte Subminiatur-Therapie-Laser dar, der ein bedingt eigensicheres Laser-System aufweist.

Für den Betrieb von Geräten der Laser-Klasse IIIB werden im medizinischen Bereich meistens folgende Anforderungen gestellt:
1.Das Gerät muß einen Schlüsselschalter aufweisen.
2.Das Gerät muß eine Emissions-Warneinrichtung haben.
3.Das Gerät muß eine Leistungskontrollanzeige haben.
4.Das Gerät darf nur von geschultem oder geprüftem Personal bedient werden.
5.Das Gerät darf nur in speziell eingerichteten und gekennzeichneten Räumen betrieben werden.
6.Die Funktion des Gerätes muß regelmäßig durch eine dazu autorisierte Person überprüft werden.
7.Bedienungspersonal und Patienten müssen Laser-Schutzbrillen tragen.

In der deutschen Patentschrift DE 2548354 C2 wird ein Laser beschrieben, dessen Modulationsblende beim abgeschalteten Gerät mit anderer Zielsetzung nur im geöffneten Zustand stehen bleibt, so daß beim Einschalten keine zusätzliche Sicherheit vorhanden ist.

Die European Patent Application mit der Publication number 0272 325 A1, entsprechend PCT/JP86/00666 stellt einen Semiconductor Laser Therapeutic Apparatus vor, der eine Mischung aus unterschiedlichen Laserstrahlen als divergentes inkohärentes Strahlenbündel mit breiterem Emissionsspektrum, das nur bei größerem Abstand ohne zusätzliche Optik für das Auge sicher unschädlich ist.

Als Sicherheitseinrichtung besteht ein Schlüsselschalter.

Zur Verkürzung der Behandlungszeit werden mehrere Laser-Sonden eingesetzt, die flexibel angeordnet sind. Dies geschieht ohne zusätzlichen Strahlenschutz lt. deutscher Offenlegung DE 3720742 A1 und US Pat. 5.150.704.

In US-Patenten 5.140 984 und 4.854.320 "Laser Healing Method and Apparatus" werden Kontroll-und Regeleinrichtungen beschrieben, die an der Laserstrablaustrittsöffnung keine Eigensicherheit des Systems ermöglichen.

In US - Pat. 5.002 051 " Method for Closing Tissue Wounds using Radiative Energy Beams" und dem US - Pat. 4.672 969 "Laser Healing Method" wird die gleiche Sicherheitsstufe dargestellt.

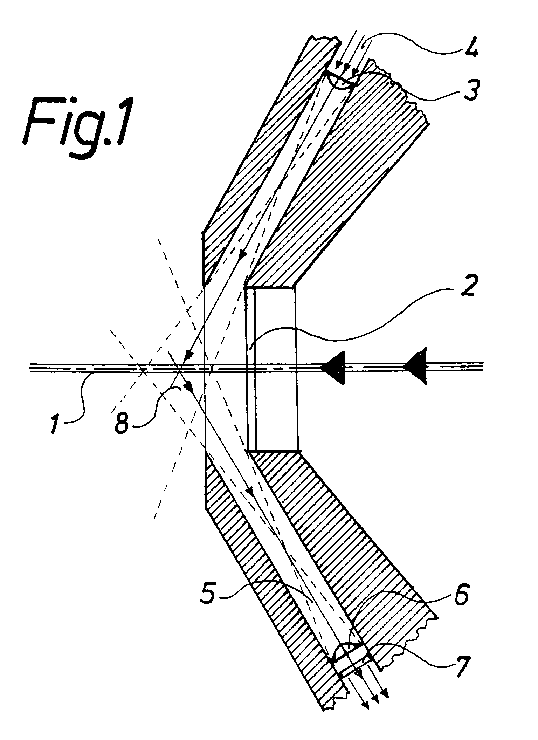

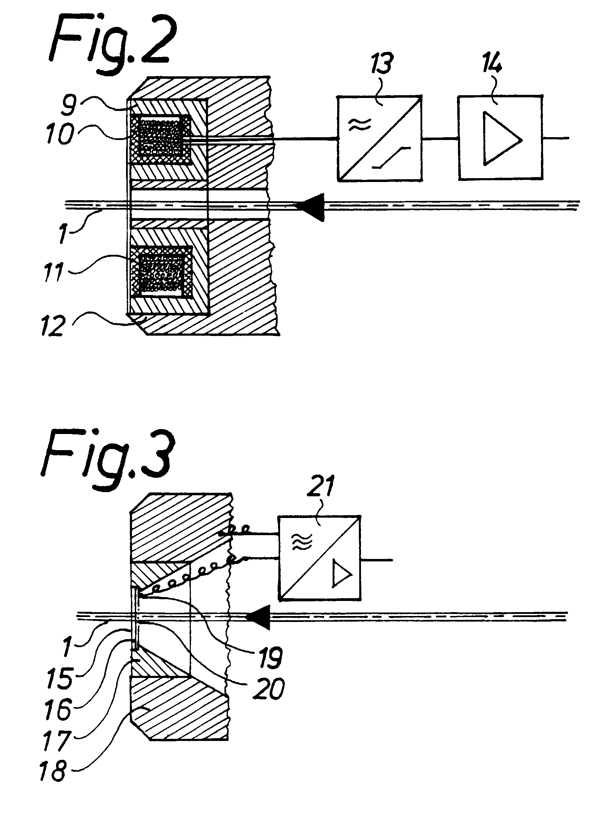

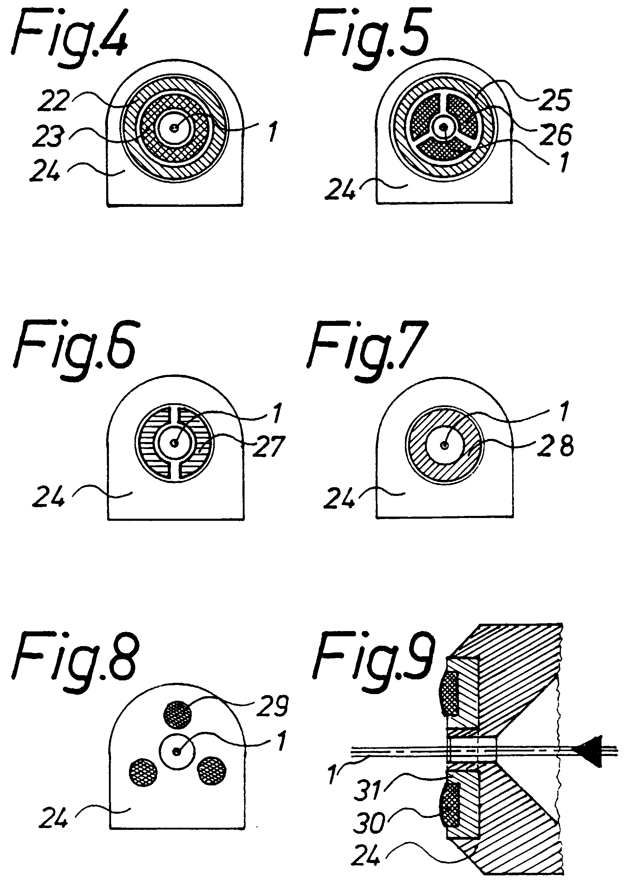

Laser-Therapie-Geräte mit vergleichbarem Sicherheitsstandard der Laserstrahlaustrittsöffnung sind in den folgenden Veröffentlichungen zu finden:
- DE Offenlegung: 2619930 (Fig.5, Laser-Endstück)
- DE Offenlegung: 2948580 (Fig.1)
- DE Offenlegung: 3719561 A1
- DE Offenlegung: 3729288 A1 (Behandlungsgriffel (4))
- DE Offenlegung: 3226507 A1 (Fig.1 (13, 14)
- EP: 0320 080 (Fig.3)
- EP: 0190 107 A2 (Fig.3)
- EP: 0429 297 A2
- EP: 0435 506 A2

In der Offenlegung DE 3545873 A1 wird ein Gerät zur Behandlung mit Laserstrahlen vorgestellt, das erfindungsgemäß eine Doppelschalteranordnung als vorteilhafte Sicherheitsmaßnahme hat.

Entsprechend EP 0292 621 A1 erfolgt die Laser-Abstrahlung als diffuser Strahlenkegel mit für das Umfeld reduzierter Gefahr. Eine große Divergenz der abgestrahlten Laserstrahlung durch lichtdurchlässige Dichtungsendabschnitte wird in der Offenlegung DE 4212393 (JP 3-51644) beschrieben.

Bei chirurgischen Lasern mit entsprechend höherer Ausgangsleistung sind die Applikation betreffend am Gerät zusätzliche Sicherheitseinrichtungen bekannt, wie z.B. in Patentschrift DE 2829 516 C2 dargestellt.

Aus US-A-5 200 604 ist ein Näherungsschalter auf optischer Basis bekannt, der neben der Strahlachse des Laserstrahles mit zweiter Lichtquelle (20; Abb.1) einen Lichtreflex-Abstandssensor hat (22).

Die Veröffentlichung DE-A-25 48 354 beschreibt einen Laser, bei dem eine Elektrode (24) mit einer Gegenelektrode mit platten- oder zylinderförmiger Ausführung, Teil eines Widerstandsmeßgerätes ist, das einen einstellbaren Greuzwertschalter enthält, dessen oberer oder unterer Grenzwert sich einstellen läßt und bei dessen Erreichen während der Behandlung sich über den Betriebsstromkreis die Lichtquelle abschalten läßt (siehe Spalte 4, Zeilen 31-57).

DE-U-86 28 810 offenbart eine gefederte Kontaktspitze, die seitlich voll zugänglich ist und bei entsprechender Andruckkraft einen elektrischen Schalter schließt und somit den Laserstrahl freigibt.

Aus US-A-5 140 984 ist ein mechanischer Verschluß bekannt, der über einen elektrischen Schaltkontakt ausgelöst werden kann.

### Aufgabe:

Laser - Therapie - und Diagnose - Geräte für den medizinischen Einsatz im unteren Leistungsbereich bis 500 mW werden entsprechend der national zum Teil unterschiedlichen Normen und Regeln zur Laser-Sicherheit meist in Laser-Klasse III eingeordnet. Dies bedeutet für den fachkundigen Anwender erhebliche Einschränkungen. Dem Laien, Patient oder Endverbraucher bleibt die freie Verfügbarkeit dieser Geräte durch entsprechende Sicherheitsauflagen verwehrt.

Ziel dieser Erfindung ist es, entsprechend den national unterschiedlichen Sicherheitsanforderungen, Laserstrahlaustrittsöffnungen so zu gestalten, daß im Leistungsbereich bis max. 500 mW eigensichere Therapie- und Diagnosegeräte entstehen, die außerhalb medizinischer Behandlungsräume oder ohne ständige Aufsicht von medizinischem Fachpersonal zur Behandlung eingesetzt werden dürfen.

Da medizinische Soft- oder Mid-Laser für die Bestrahlung von biologischen Zellen und Geweben eingesetzt werden und bei den verwendeten Energiedichten und möglichen Expositionszeiten nach den wissenschaftlichen Erkenntnissen kein Hautschutz bei Mensch und Tier erforderlich ist, muß sich die Sicherheitstechnik auf den Augenschutz beschränken. Es muß weiteres Ziel der Erfindung sein Laserstrahlaustrittsöffnungen vorzustellen, die eine Bestrahlung von Haut, organischen Zellen und Geweben ermöglichen, bei zugleich absoluter Augen-Strahlungssicherheit. Ein konstruktiver Schritt in diese Richtung wurde bereits bei dem "Subminiatur - Therapie - Lasergerät zur Biostimulation von organischem Gewebe" EP 0416 150 A1 gezeigt. Eine an der Austrittsöffnung beginnende große Divergenz des Laserstrahles verbunden mit einer Einschaltsperre, die von einem Sensorring um die Strahlaustrittsöffnung angesteuert wird, garantiert eine gewisse Eigensicherheit des Systems.

Als nachteilig hat sich hierbei herausgestellt, daß der Sensorkontakt nach Fig.1 (7) nicht täuschungssicher ist, d.h. eine einseitige seitliche Berührung mit nur einer Fingerspitze reicht aus, um die Einschaltsperre aus und den Laser einzuschalten, obgleich die Laserstrahlaustrittsöffnung für die Umwelt freigegeben ist!

### 3.)Darstellung der Erfindung

Die Erfindung, wie sie in den Ansprüchen gekennzeichnet ist, löst die Aufgabe dadurch, daß Laser-Therapie- und Diagnosegeräte im Leistungsbereich der Mid- und Soft-Laser mit einer Sicherheits- Laserstrablaustrittsöffnung (SLA) versehen werden, so daß sie entsprechend der national unterschiedlich strengen Laser-Sicherheitsbestimmungen als eigensichere Systeme in Bereichen außerhalb medizinischer Behandlungsräume und/oder ohne ständige Aufsicht von medizinischem Fachpersonal eingesetzt werden dürfen.

Die nach medizinischer Indikation und Anweisung mögliche nahezu autonome Behandlung wird sich hauptsächlich auf die in Anspruch 1 genannten Bereiche und Krankheiten erstrecken. Literaturangaben hierzu sind in EP 0416 150 A1 zum Stand der Technik angegeben.

Entsprechend der Ansprüche 2 bis 10 gibt es in Verbindung mit nationalen Sicherheits-Regeln verschiedene Wege zur Ausführung der Erfindung.

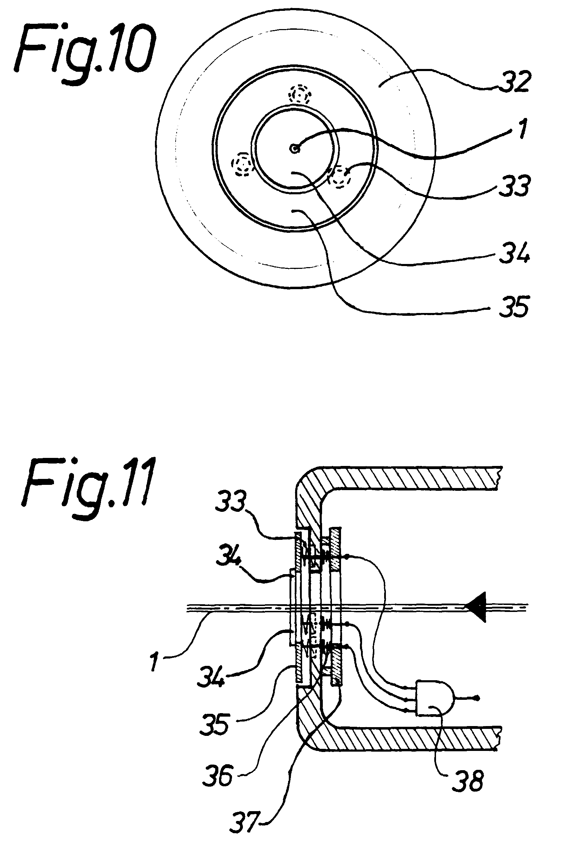

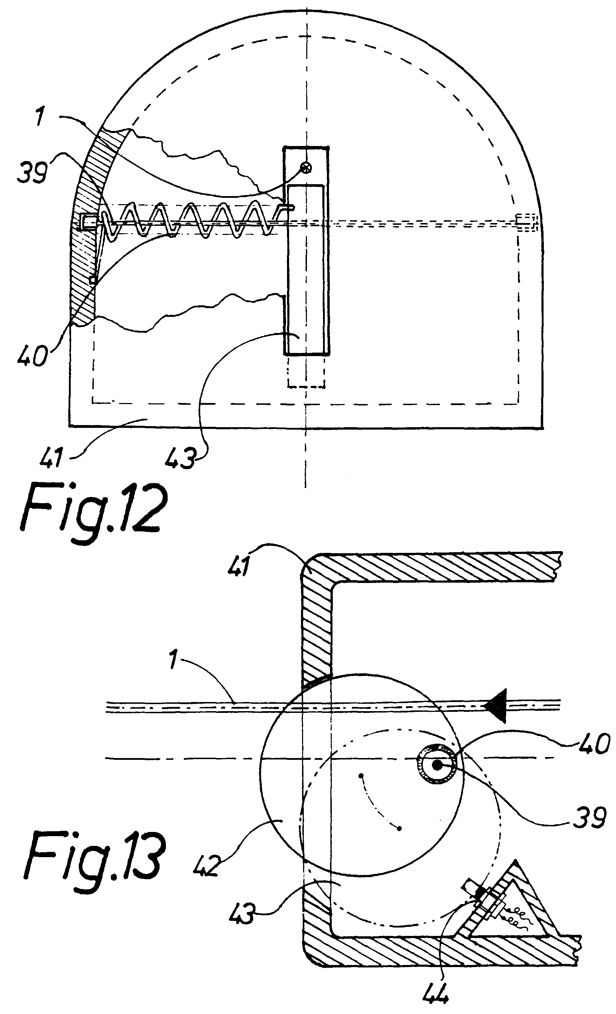

Allen Wegen gemeinsam ist das Kennzeichen der Erfindung, daß die Sicherheits-Laserstrahlaustrittsöffnungen (SLA) mit einer großen Täuschungssicherheit, bevorzugt mit organischen Zellen oder Zellverbänden, menschlicher- oder tierischer Haut bedeckt werden müssen, um die Einschalt- und Betriebssperre des Laser-Gerätes zu lösen und den Laserstrahl zugleich am freien Austritt in den Raum zu hindern, d.h. ihn nur für die Bestrahlung des organischen Materials zuzulassen. Erfindungsgemäß wird dies durch folgende technischen Vorrichtungen möglich:
I.SLA mit Lichtreflex-Abstands-Sensor (LAS) Eine SLA mit LAS (Fig.1) ist so konstruiert, daß die Laserstrahlaustrittsöffnung mit einem Gegenstand, vorzüglich organischem Gewebe oder Haut abgedeckt werden muß, um einen auf diesen Bereich gerichteten, für das menschliche Auge absolut ungefährlichen, schwachen Licht- oder Laserstrahl in Richtung Laser-Gerät zu reflektieren, um dann von einem Licht-Sensor erfaßt zu werden und die Einschalt- und Betriebssperre des Lasers für die Zeit der Abdeckung zu lösen.
   Eine spezifische Erkennung des abdeckenden Materials wird möglich durch verschiedene Filtereinrichtungen für das reflektierte Licht. Manipulationssicherheit kann durch kodierte Modulation des Lichtstrahles erreicht werden.
   Vorteile:
   1.Erschütterungsunempfindlichkeit
   2.Langlebigkeit
   3.Keine Betätigungskraft erforderlich
   4.Durch Einsatz von Lichtleitfasern ist eine große Flexibilität des Systems möglich, z.B. dünne Behandlungsgriffel
   5.Große Sicherheit der SLA und diagnostische Verwertbarkeit des reflektierten Lichtstrahles durch spektroskopische Auswertungsmöglichkeit bei gleichzeitig möglicher Therapieüberwachung und Materialerkennung
      (Es sind spektroskopische Veränderungen in organischen Zellen und Geweben bei Einwirkung von Laserstrahlen bekannt, sowie auch in kranken Organismen.)
   6.Keine mechanisch bewegten Teile
II.SLA mit Näherungsschalter, der induktiv, kapazitiv oder hochfrequent das sich ihm dicht nähernde Material erkennt und für die Zeit der dichten Annäherung die Betriebs- und Einschaltsperre des Lasers ausschaltet.
   Bei dem induktiven Näherungsschalter (Fig.2) läßt sich entsprechend der Kurzbeschreibungen der Zeichnungen mit geringem Aufwand die erforderliche Spule (10, 11) um die Laserstrahlaustrittsöffnung anordnen. Nachteilig ist bei den Näherungsschaltern, daß sie mit zusätzlichen schaltungstechnischen Einrichtungen versehen werden sollten, damit sich verschiedene im Baum befindliche Näherungsschalter nicht gegenseitig beeinflussen können.
   Es kann der Schaltabstand bei verschiedenen auslösenden Materialien und in Abhängigkeit von der Temperatur und der Luftfeuchtigkeit variieren.
   Der kapazitive Näherungsschalter arbeitet mit einem R-C-Schwingkreis, bei dem die Kapazität (C) beeinflußt wird.
   Dies geschieht durch Annäherung des zu erfassenden Materials an die aktive Fläche der einen Elektrode Fig.3 (16), Fig.6 (27), Fig.7(28). Die zweite Elektrode ist entweder das zu erfassende Material mit der Masse als Rückleitung Fig.3 (18), Fig.7 (24) oder ein zweiter Elektrodenteil Fig.6 (27 gegenüberliegender Elektrodenteil) wobei das zu erfassende Material eine Änderung des Dielektrikums bewirkt. Nähert sich das zu erfassende Material der SLA und somit der Kondensatorelektrode des Näherungsschalters, so erhöht sich die Kapazität soweit, bis der Wert der Abstimmung mit dem Widerstand (R) im Schwingkreis erreicht ist und der Oszillator zu schwingen beginnt ( < 200 MHz) wodurch die Einschalt- und Betriebssperre des Lasers ausgeschaltet wird. Entsprechend EP 0136 530 (A61N 5/02) ist das abgestrahlte hochfrequente elektromagnetische Feld therapeutisch nutzbar. Die Hochfrequenz läßt sich auch zur Modulation des Lasers verwenden. Dies kann therapeutisch sinnvoll sein.
   Vorteile:
   1.Ohne Betätigungskraft schaltbar
   2.Keine mechanisch bewegten Teile
   3.Unempfindlich gegen mechanische Erschütterungen
   4.Große Lebensdauer
   5.Möglichkeit die Hochfrequenz therapeutisch in Kombination mit dem Laser zu nutzen
      a) zur Modulation des Lasers
      b) als abgestrahltes hochfrequentes elektromagnetisches Feld.
III. SLA mit Sensorkontakten,
   die auf die Leitfähigkeit des sie berührenden Materials ansprechen und nur bei gemeinsamer Berührung aller Sensorkontakte (mindestens 2 Stück) Fig.4, Fig.5, Fig.6 durch das auslösende Material die Einschalt-und Betriebssperre des Lasers für die Zeit der Berührung lösen.
   Um eine zusätzliche Sicherheit vor Manipulation und Täuschung zu gewährleisten, ist es erforderlich mindestens zwei Elektroden auf der Außenseite der SLA als Sensorkontakte zu befestigen. Die Elektroden können ringförmig geschlossen oder ringförmig segmentiert nebeneinander angeordnet sein.Die Bezugselektrode kann für die Eigentherapie in Form einer leitfähigen Gehäuseoberfläche Fig. 4 - 6 (24) oder als äußere Ringelektrode Fig. 4+5 (22, 25) ausgebildet sein, bevorzugt für die Fremdtherapie. Zwischen der Bezugselektrode und den Sensorkontakten fließt ein vom Widerstand des berührenden Materials und der anliegenden Spannung abhängiger Gleich- oder Wechselstrom, der zur Erkennung des berührenden Materials dient.
   Nur während der Berührung aller Sensorkontakte wird über eine logische UND-Verbindung die Einschalt- und Betriebssperre des Lasers ausgeschaltet. Die isoliert in der SLA eingebetteten Elektroden (Sensorkontakte) weisen vorteilhaft eine leichte Wölbung zur Außenseite hin auf Fig.9 (30).Dies bewirkt im Bereich der Elektroden leicht erhöhte Andruckkräfte und somit einen verbesserten Kontakt zum berührenden Material.
   Die Sensorkontakte oder ihre Oberfläche sollten aus gut leitendem, wenig korrodierendem Material sein, um die Ausfallrate der Sicherheitseinrichtung gering zu halten, wobei natürlich ein fehlerhafter Kontakt keine geringere Sicherheit der SLA bedeutet, vielmehr nur einen Ausfall des Lasers. Das Kontaktmaterial sollte zugleich keimhemmende oder keimtötende Eigenschaften aufweisen, bei gefordertem geringem allergenen Potential. Dies ist im Hinblick auf den in Anspruch 1 genannten Anwendungsbereich als zusätzliche hygienische Sicherheit empfohlen, wenn die Geräte von verschiedenen Personen in dichten Abständen benutzt werden. Hierzu haben sich Materialien aus Silber, Kupfer, Gold und Platin und deren Verbindungen erfindungsgemäß gut bewährt.
   Vorteile:
   1.Einfache und kostengünstige Konstruktion
   2.Gute Störsicherheit
   3.Nichtleitende Materialien werden nicht erfaßt
   4.Annäherung des Materials reicht nicht für den Einschaltvorgang aus (kann auch Nachteil sein, je nach Einsatz des Gerätes, z.B. bei der Behandlung von Ulcus cruris)
   5.Sehr geringe Andruckkräfte bei der Berührung reichen für den geforderten Schaltvorgang aus.
IV. SLA mit Sensorkontakten, die auf Andruckkräfte des sie berührenden Materials ansprechen.
   Entsprechend der Kurzbeschreibung zu Fig.10 und Fig.11 ergibt sich die konstruktive Möglichkeit, über eine Andruckplatte (35) von außen mit leichter Andruckkraft durch das am Austrittsfenster (34) anliegende Material einen oder mehrere in Serie geschaltete mechanische Kontakte einzuschalten, die als Einschalt- und Betriebssperre für den Laser dienen.
   Vorteile:
   1.Einfache mechanische Konstruktion
   2.Große elektromagnetische Verträglichkeit (störsicher)

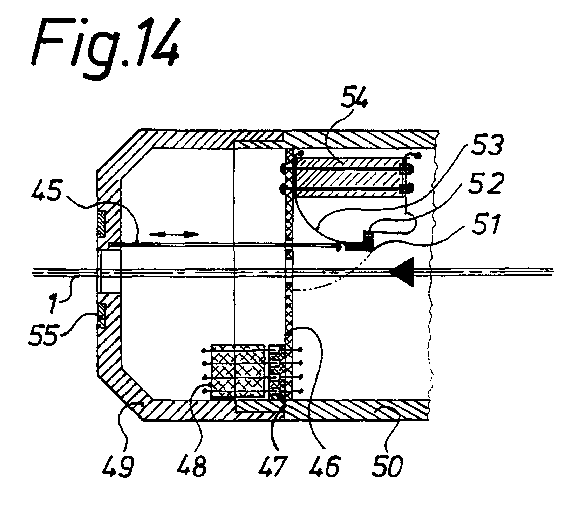

   Die von Folien-Tastaturen,-Potentiometern und Touch-Screens hinreichend bekannten leitfähigen Folienelemente lassen sich erfindungsgemäß als Drucksensoren wie auch Elemente aus Halbleiterpolymer-Folien Fig.6, 7 und 8 ebenso einsetzen, wie piezoxide Materialien und Sensoren, die mit Dehnungsmeßstreifen versehen sind oder nach diesem Prinzip bei leichtem Andruck durch das berührende Material die Einschalt- und Betriebssperre des Lasers ausschalten.
   Vorteile:
   1.Unempfindlich gegen Verschmutzung
   2.Wasserdicht und chemikalienbeständig
   3.Hohe Lebensdauer
   4.Geringerer Berührungsdruck als bei der mechanischen Andruckplatte
   5.Höhere Sicherheit durch größere Anzahl von in Serie schaltbaren Kontakten (bei Einsatz von Elementen nach dem Prinzip der Folientastaturen) möglich
V. SLA mit mechanischem Verschluß.
   Entsprechend der Kurzbeschreibung von Fig.12 und Fig.13 dargestellte mechanische Vorrichtung, deren Exzenterscheibe (42) durch Andruck des berührenden Materials von außen den in Ruhestellung mechanisch verschlossenen Laserstrahlengang freigibt und zugleich einen elektrischen Kontakt schließt, der die Einschalt- und Betriebssperre ausschaltet. Es sind viele verschiedene mechanische Sicherheitsverschlüsse möglich. Die aufgezeigte Version stellt einen fertigungstechnisch einfach und kostengünstig herstellbaren Verschluß dar, der wenig störanfällig und somit sicher ist.
VI. Sicherheits-Service-Verschluß der SLA
   Um die verschiedenen Ausführungsarten der SLA zu reinigen oder zu desinfizieren ist es erfindungsgemäß zweckmäßig das Laser-Therapie- oder -Diagnosegerät auch dann in einem eigensicheren Zustand zu erhalten, wenn die SLA vom Gerät entfernt wird. Dies geschieht beispielhaft entsprechend Fig. 14 und der Kurzbeschreibung dazu.
   Die Steckverbindung (47, 48) stellt für den Betrieb der SLA kein Sicherheitsrisiko dar, da jede stromführende Leitung zu allen hier vorgestellten Sensoren bei ihrer Unterbrechung ein Einschalten und /oder Betreiben des Lasers unmöglich macht.

### 4.) Kurzbeschreibung der Zeichnungen

Fig.1
   Sicherheits-Laserstrahlaustrittsöffnung (SLA) mit Lichtreflex-Abstands-Sensor.
   Das von einer Lichtquelle emittierte Licht (4), das elektronisch mit einer Kodierung versehen sein kann, wird von einer Abschlußoptik (3), die in Form einer Sammellinse gestaltet werden kann, über den sich anschließenden reflexionsfrei beschichteten Strahlenkanal in den reflektierenden Bereich (8) unmittelbar vor der SLA abgestrahlt.
   Befindet sich, z.B. Haut als zu bestrahlende Materie in dem reflektierenden Bereich (8), so wird ein Teil der Lichtstrahlung reflektiert und weitergeleitet über den gegenüberliegenden antireflexbeschichteten Strahlenkanal (5) zur aufnehmenden Abschlußoptik (6). Von hier gelangt das Licht über eine mögliche Filterscheibe (7) zur Empfangs- und Auswertelektronik, die die Einschalt- und Betriebssperre des Lasers ausschaltet. Das Strahlaustrittsfenster (2) ist zur Vermeidung von unerwünschten Reflexionen vor dem reflektierenden Bereich (8) angeordnet.
Fig.2
   SLA mit induktivem Näherungsschalter.
   Der Oszillator (13) des induktiven Näherungsschalters erzeugt mit Hilfe der im offenen Schalenkern (9) liegenden Spule (10, 11) ein höher frequentes, magnetisches Wechselfeld ( ≦ 500 Hz), das zur Außenseite der SLA austritt. Wird in dieses Feld ein elektrisch leitendes Material, z.B. organisches Gewebe gebracht, so entsteht eine Induktionswirbelspannung. Der fließende Wirbeistrom entzieht dem L-C-Schwingkreis Energie. Die Belastung des Oszillator - Schwingkreises bewirkt eine Verkleinerung der Schwingungsamplitude. Die Verkleinerung der Amplitude wird von dem nachgeschalteten Verstärker (14) in ein eindeutiges elektrisches Signal umgewandelt und schaltet die Einschalt- und Betriebssperre der SLA aus.
   Die Anordnung ist in das Gehäuse (12) der Laserstrahl-Austrittsöffnung eingebettet.
Fig.3
   SLA mit kapazitivem- oder Hochfrequenz - Näherungsschalter.
   Das Laseraustrittsfenster (15) ist isoliert im Gehäuse (18) eingebettet (17). Die Rückseite des Laseraustrittsfensters (16) kann durchgehend nach dem Prinzip der Kathodenzerstäubung (Sputtering) mit einer glasklaren gleichmäßigen Beschichtung von Metallen, wie z.B. Nirostastahl, Silber, Gold und Titan beschichtet werden, so daß über das gesamte Austrittsfenster eine elektrisch gut leitende Schicht entsteht, die zugleich eine hohe Transmissionsrate für den Laserstrahl (1) zuläßt. Diese Beschichtung dient als Elektrode für die Auswertelektronik (21). Der Übergang zur Zuleitung wird durch eine ringförmige Silberbeschichtung (19) erreicht, so daß der innere Bereich (20) um den Laserstrahl (1) für die Transmission frei bleibt.
Fig.4
   SLA mit Anordnung der Sensorkontakte zur Auswertung der Leitfähigkeit des sie berührenden Materials.
   Der austretende Laserstrahl (1) wird in möglichst geringem Abstand von einer Ringelektrode (23) umgeben, die so wie auch die umgebende Gegenelektrode (22) nach dem Verfahren in Fig.9 (30) isoliert (31) außen eingebettet ist. Die Anordnung ist auch für den kapazitiven Näherungsschalter verwendbar.
Fig.5
   SLA mit Anordnung von mehreren Sensorkontakten zur Auswertung der Leitfähigkeit des sie berührenden Materials.
   Der austretende Laserstrahl (1) wird in möglichst geringem Abstand von drei ringförmig voneinander unabhängigen Elektroden (26) umgeben, die an der SLA außen angeordnet sind. Zu den drei Elektroden gibt es eine besondere gemeinsame ringförmige Gegenelektrode (25) oder das Gehäuse (24).
Fig.6
   SLA mit Anordnung von zwei Sensorkontakten zur Auswertung der Leitfähigkeit des sie berührenden Materials oder als isolierte Kontakte für einen kapazitiven Näherungsschalter.
   Die Anordnung der Elektroden (27) kann sowohl zur Auswertung der Leitfähigkeit als auch für den kapazitiven Näherungsschalter oder den Drucksensor verwendet werden.
Fig.7
   SLA mit einfacher um den Laserstrahl (1) ringförmiger Anordnung des Sensorkontaktes (28) als entweder Drucksensorkontakt oder Einzelsensor für kapazitive oder Hochfrequenz-Näherungsschalter.
Fig.8
   SLA mit dreigeteilten, möglichst dicht um den Laserstrahl (1) angeordneten Drucksensorkontakten (29).
Fig.9
   Schnitt der Ausführung und Einbettung von gewölbten Sensorkontakten (30) in die möglichen Gehäuse der SLA (24) mit isolierenden Werkstoffen (31).
Fig.10
   Vorderansicht der SLA mit mechanischer Andruckplatte.
   Im Gehäuse der SLA (32), zentrisch um den Laserstrahl (1) angeordnet, befindet sich federnd (33) gegen das Gehäuse (32) mit dem Austrittsfenster (34) fest verbunden eine Andruckplatte.
Fig.11
   Seitlicher Schnitt der SLA nach Fig.10 mit mechanischer Andruckplatte.
   Die federnd (33) aufgehängte Andruckplatte (35) wird durch leichten Andruck über dem Laserstrahlaustrittsfenster (34) in Richtung der fest mit dem Gehäuse verbundenen Gegenplatte (37) soweit von dem außen anliegenden Material bewegt bis sich die Kontakte (36) schließen.
   Die drei Kontakte (36) müssen gemeinsam geschlossen sein, um die Einschalt- und Betriebssperre des Lasers zu lösen. Dies geschieht über eine Serienschaltung der drei Kontakte oder über eine logische UND-Verbindung (38).
Fig.12
   SLA mit mechanischem Verschluß.
   Ein im Gehäuse (41) über eine Welle (39) drehbar gelagerter Exzenter (43) wird über eine leichte Kraft von außen durch das berührende Material gegen den Widerstand der Spiralfeder (40) so nach innen bewegt, daß der Laserstrahl (1) eine Öffnung nach außen erhält.
Fig.13
   Seitlicher Schnitt der SLA mit mechanischem Verschluß nach Fig.12.
   Ein in Ruhestellung, bewirkt durch die Spiralfeder (40), den Strahlengang des Lasers völlig verschließender Exzenter (42), wird durch leichten Andruck von außen, besonders durch menschliche oder tierische Hautoberflächen in die Betriebsstellung (43) gebracht, in der er den Strahlengang freigibt und zugleich den Schalter (44) zum Lösen der Einschalt- und Betriebssperre betätigt.
   Mit der Entfernung des berührenden Materials wird die Laserstrahlaustrittsöffnung wieder sicher verschlossen (42).
Fig.14
   Seitlicher Schnitt der SLA mit Sicherheits-Service-Verschluß.
   Beim Entfernen der SLA vom Lasergerät wird der vordere Gehäuseteil (49) vom Hauptgehäuse (50) abgezogen. Mit dem vorderen Gehäuseteil (49) fest verbunden sind der Schlüsselstift (45) und der Steckersockel (48). Der Schlüsselstift (45) wird dabei durch die Öffnung in der Trennwand (46) zurückgezogen, was einen unmittelbaren Verschluß des Strahlenkanals an der Öffnung in der Trennwand (46) durch das federnd (53) aufgehängte Verschlußteil (51) bewirkt und zugleich die Einschalt- und Betriebssperre durch den geöffneten Kontakt (52) an der Rückseite des Verschlußteils (51) einschaltet.
   Zugleich werden beliebig viele Kontakte der Steckverbindung (47) und (48) gelöst. Der gesamte Vorgang ist reversibel.

### 5.)Wege zur Ausführung der Erfindung

Die in der Darstellung der Erfindung aufgezeigten Sicherheits-Laserstrahlaustrittsöffriungen (SLA) mit verschiedenen Sicherheitsanordnungen (Fig. 1 - 14) werden entsprechend unterschiedlich zum Einsatz kommen in Abhängigkeit der:
1.Unterschiedlichen nationalen Sicherheitsanforderungen
2.Konstruktiven Merkmalen des eingesetzten Laser-Therapie- und -Diagnosegerätes
3.Kostenfaktoren bei der industriellen Fertigung der unterschiedlichen Sicherheitsanordnungen für die jeweilige SLA
4.Therapeutischen oder diagnostischen Verwertbarkeit der durch die SLA gewonnenen oder erzeugten Parameter, z.B. 1. Lichtreflex-Abstands-Sensor (fotometrische Auswertung des Signals), 2. SLA mit Näherungsschalter (Magnetwechselfelder oder Hochfrequenz sind zur Modulation des Lasers oder für die Therapie und Diagnose nutzbar), 3. Sensorkontakte, die auf die Leitfähigkeit reagieren (aufspüren von pathologischen Hautwiderstandsbereichen und ihre Veränderung).

Die Einsatzchancen sind für alle vorgestellten SLA-Versionen und eine Kombination daraus gleich gut, ausgenommen die mechanischen Berührungskontakte (Fig. 10 und 11) entsprechend der Darstellung, die durch Drucksensoren kostengünstiger ersetzt werden können.

Alle Vorrichtungen verfügen über eine Einschaltkontrolle.
1. Die SLA mit Lichtreflex-Abstands-Sensor entsprechend der Darstellung der Erfindung wird vorteilhaft mit einer zweifarbigen Leuchtdiode (rot/grün) (wenn verfügbar - mehrfarbig!) als Lichtquelle ausgestattet, um ein breiteres Spektrum für die fotometrische Auswertung des reflektierten Strahles zur Verfügung zu haben. Zur Codierung wird die zweifarbige Leuchtdiode amplitudenmoduliert, so daß die rote und grüne Phase sich abwechseln. Die Elektronik auf der Seite des Sensors, der durch eine Zweifach-Fotodiode mit jeweils unterschiedlicher spektraler Empfindlichkeit gebildet wird, wertet die unterschiedlichen Signale aus für die Ansteuerung der Einschalt- und Betriebssperre, sowie zur fotometrischen Erfassung der Materialeigenschaften und -Veränderungen.
   Die beschriebene Ausführung eignet sich auch gut für Bestrahlungsgeräte im angegebenen Leistungsbereich zur Bestrahlung von Zellkulturen.
2. SLA mit Näherungsschalter wird als kapazitiver Näherungsschalter ausgeführt, so wie er beschrieben ist nach Fig.3. Es wird eine Frequenz von 150 MHz eingesetzt, die zugleich therapeutisch bei Mensch und Tier nutzbar ist. Das Strahlaustrittsfenster ist von der Rückseite mittels Kathodenzerstäubung mit einer dünnen Metallschicht versehen, die leitfähig ist und für Licht eine gute Transmission aufweist. Dadurch wird erreicht, daß das sich dicht annähernde Material sich unmittelbar vor der Strahlaustrittsöffnung befinden muß, um den Einschalt- oder Betriebszustand zu ermöglichen.
   Diese SLA nach Anspruch 2 und 3 werden da eingesetzt, wo eine unmittelbare Berührung mit der zu behandelnden Materie nachteilig ist, z.B. bei offenen Geschwüren, im Mund- und Rachenraum bei Mensch und Tier, bei organischen Zellen und Zellkulturen.
3. SLA mit Sensorkontakten, die auf die Leitfähigkeit ansprechen.
   Wie entsprechend dargestellt wird hier eine Elektrodenanordnung nach Fig.5 (26) angewandt, wobei die äußere Ringelektrode (25) entfällt und als Gegenelektrode das leitfähige Gehäuse verwendet wird. Die Elektroden (26) sind nach Fig.9 (30) gestaltet. Durch die Verwendung von drei Elektroden mit nachgeschaltetem UND-Gatter und Auswertelektronik, die Überbrückungswiderstände ≦ 25Ω als Falschsignal interpretiert, wird eine Täuschung, z.B. durch Überbrückung der Elektroden, nur unter Laborbedingungen möglich.
   Zugleich ist bei drei Elektroden noch eine ausreichend gleichmäßige Andruckkraftverteilung durch das anliegende Material und mithin ein hinreichender Übergangswiderstand möglich, was mit zunehmender Elektrodenzahl reduziert wird. Das Elektrodenmaterial ist Silber.
4. SLA mit Sensorkontakten, die auf Andruckkräfte ansprechen.
   Wie in der Darstellung gezeigt soll hier als beste Möglichkeit das Prinzip der Folientastaturen mit Halbleiterpolymerfolie nach der Anordnung von Fig.7 (28) mit einer internen Unterteilung im Folienelement nach Fig.6 (27) erfolgen. Die Unterteilung ist erforderlich, damit das System nicht so leicht durch seitlichen Fingerandruck getäuscht werden kann. Es werden hochempfindliche Elemente verwendet, die nur geringe Andruckkräfte zum Schalten der beiden über UND-Gatter verbundenen Anschlüsse benötigen.
   Der Einsatz von Elementen mit Dehnungsmeßstreifen oder piezoxiden Elementen erfordert mehr Aufwand bei der elektronischen Schaltung und hat einen größeren Kostenaufwand.
5. SLA mit mechanischem Verschluß
   Diese Ausführung wird bestmöglich nach der Kurzbeschreibung von Fig. 12 und 13, sowie der Darstellung ausgeführt.
   Damit der Exzenter eine geringe Masse aufweist (geringe Trägheit) ist er als Aluminiumgußteil im Inneren gitterförmig ausgestaltet. Die äußere Oberfläche ist mit Silber beschichtet.
6. Sicherheits-Service-Verschluß der SLA.
   Wie in der Darstellung der Erfindung und in der Kurzbeschreibung der Zeichnungen Fig.14 ausgeführt. Die Trennwand (46) ist aus isolierendem Kunststoff hergestellt.
6.)Gewerbliche Anwendbarkeit
   Mid- und Softlasergeräte werden weltweit industriell hergestellt und entsprechend der unterschiedlich strengen nationalen Sicherheitsvorschriften eingesetzt. Durch die Erfindung der Sicherheits-Laserstrahlaustrittsöffnungen (SLA) werden die mit der SLA versehenen Laser-Geräte eigensicher und der Einsatz der Geräte wird auf eine wesentlich breitere Basis gestellt. Die Geräte können somit in wesentlich größerem Umfang als bisher produziert werden.

## Patentansprüche

1. Sicherheits - Laserstrahlaustrittsöffnungen (SLA) für medizinische Therapie- und Diagnosegeräte im Leistungsbereich der MID- und SOFT-Laser für besonders hohe Sicherheitsanforderungen, dadurch gekennzeichnet, daß die Geräte, die mit den Sicherheits - Laserstrahlaustrittsöffnungen versehen sind, mit großer Täuschungs- und Manipulationssicherheit als eigensichere Systeme, entsprechend national unterschiedlich strenger Sicherheitsbestimmungen,den Einsatz außerhalb medizinischer Behandlungsräume und/oder autonom ohne ständige Aufsicht von medizinischem Fachpersonal erlauben,
von außen bevorzugt mit organischen Zellen oder Zellverbänden menschlicher oder tierischer Haut bedeckt werden müssen, um nur für diese Zeit den Versorgungsstrom des Lasers mit Hilfe der nachfolgend wahlweisen Merkmale a) bis f) einzuschalten, bzw. die Einschalt- und Betriebssperre des Lasers zu lösen und zugleich den Laserstrahl am freien Austritt in den Raum zu hindern und eines oder mehrere der folgenden Merkmale haben,
a) einen im direkten Strahlungsbereich des Laserstrahles mit zweiter Lichtquelle detektierender Lichtreflex-Abstandssensor (2,3,5,6,7,8)
und/oder
b) einen hochfrequenten (1,15,16,17,18,19,20,21,24,28) oder induktiven (9,10,11,12,13,14) oder kapazitiven (1,15,16,17,18,19,20,21,24,27,28) Näherungsschalzer
und/oder
c) mindestens zwei Sensorkontakte (1,22,23,24,25,26,27), die auf den elektrischen Widerstand des sie berührenden Materials ansprechen
und/oder
d) mindestens einen Sensorkontakt randwärts nach außen um die SLA angeordnet (1,24,27,28,29), der materialbedingt auf die Andruckkräfte des berührenden Materials elektrisch anspricht
und/oder
e) drei über eine Andruckplatte verbundene Sensorkontakte (1,32,33,34,35,36,37), die über ein UND-Gatter (38) den gemeinsamen Schaltkontakt auslösen
und/oder
f) einen mechanischen Verschluß der Austrittsöffnung (1,39, 40,41,42,43,44) mit elektrischem Schaltkontakt.

2. Sicherheits-Laserstrahlaustrittsöffnungen nach Anspruch 1, dadurch gekennzeichnet, daß die SLA mindestens einen Lichtreflex-Abstands-Sensor, LAS, haben, dessen emittierter Lichtstrahl von seiner Strahlungsquelle (4) aus durch einen strahlbegrenzenden und mit der Möglichkeit der Antireflexbeschichtung versehenen Strahlenkanal seitlich zwischen Laserstrahlaustrittsfenster und äußerem, die SLA begrenzenden Rand, so auf die Achse des austretenden Laserstrahles gerichtet ist, daß es diesen unmittelbar vor der SLA schneidet und in diesem Bereich (8) sich das von dem Laser zu bestrahlende Material zwingend befinden muß, um von der, im ebenfalls mit der Möglichkeit der Antireflexbeschichtung versehenen Strahlenkanal (5), seitlich versetzt angeordneten aufnehmenden und mit einem möglichen Filter (7) versehenen Abschlußoptik (6) erfaßt und mittels Sensor zum Ausschalten der Einschaltsperre des Lasergerätes zu dienen, bei gleichzeitig möglicher fotometrischer Materialbestimmung.

3. Sicherheits-Laserstrahlaustrittsöffnungen nach Anspruch 1, dadurch gekennzeichnet, daß die SLA um die Laserstrahlaustrittsöffnung nach außen einen Näherungsschalter aufweisen, der induktiv (9,10,11,12,13,14), kapazitiv (1,15, 16,17,18,19,20,21,24,27,28) oder hochfrequent (1,15,16,17, 18,19,20,21,24,28) das sich ihm dicht nähernde Material erkennt und für die Zeit der dichten Annäherung die Betriebs- und Einschaltsperre des Lasers ausschaltet und dessen Hochfrequenz zur Modulation des Lasers oder als therapeutisch nutzbare elektromagnetische Schwingungen eingesetzt werden können.

4. Sicherheits-Laserstrahlaustrittsöffnungen nach Anspruch 1, dadurch gekennzeichnet, daß die SLA mindestens zwei um die Laserstrahl-Austrittsöffnungen nach außen angeordnete Sensorkontakte (1,22,23,24,25,26,27) aufweist, die auf die Leitfähigkeit des sie berührenden Materials ansprechen und nur bei gemeinsamer Berührung aller Sensorkontakte durch das sie berührende Material die Einschalt- und Betriebssperre des Lasers für die Zeit der Berührung lösen.

5. Sicherheits-Laserstrahlaustrittsöffnungen nach Anspruch 4, dadurch gekennzeichnet, daß die Sensorkontakte zur Berührungsfläche nach außen eine leichte Wölbung (30) und seitlich eine elektrische Isolation (31) aufweisen.

6. Sicherheits-Laserstrahlaustrittsöffnungen nach Anspruch 1, dadurch gekennzeichnet, daß die SLA um die Laserstrahlaustrittsöffnungen nach außen einen oder mehrere Sensorkontakt(e) aufweis(t)(en) (1,24,27,28,29), der (die) unter Verwendung von Halbleiterpolymer-Folien oder piezoxiden Materialien oder Dehnungsmeßstreifen hergestellt ist (sind) und der (die) auf die Andruckkräfte des ihn (sie) berührenden Materials direkt anspricht (ansprechen) und bei dem eingestellten Andruck für die Zeit des Andrucks durch das ihn (sie) berührende Material die Einschalt- und Betriebssperre des Lasers ausschalte(t)(n).

7. Sicherheits-Laserstrahlaustrittsöffnungen nach Anspruch 1, dadurch gekennzeichnet, daß die SLA einen von dem den Strahlengang des Lasers verschließenden Material mit mechanischem Andruck auslösenden mechanischen Verschluß der Laserstrahlaustrittsöffnung enthält, der als Exzenter (43) mit seiner drehbar gelagerten Achse (39) quer zur Achse des Laserstrahles seitlich versetzt angeordnet eigensicher,nur bei Betätigung durch das zu bestrahlende Material,den Strahlengang des Lasers freigibt und zugleich über einen elektrischen Kontakt (44) die Einschalt- und Betriebssperre des Lasers ausschaltet und bei Entfernung des zu bestrahlenden Materials von der SLA den mechanischen Verschluß mittels einer Spiralfeder (40) selbstätig schließt und die Einschaltsperre für den Laser mit einem Schalter (44) einschaltet.

8. Sicherheits-Laserstrahlaustrittsöffnungen nach den Ansprüchen 4 + 5 + 6 + 7, dadurch gekennzeichnet, daß die äußere Berührungsfläche der Sensoren oder des mechanischen Verschlusses aus Material mit keimhemmenden- oder keimtötenden-, elektrisch gut leitenden-, sowie vor Korrosion schützenden Eigenschaften besteht, bei zugleich geringem allergenen Potential, wie Silber, Kupfer, Gold, Platin und deren Verbindungen.

9. Sicherheits-Laserstrahlaustrittsöffnungen nach Anspruch 1, dadurch gekennzeichnet, daß beim Entfernen der SLA einschließlich ihrer Sensor- und Kontaktflächen (49) vom Lasergerät (50) die Stromzufuhr des Lasers zwingend unterbrochen wird durch einen von außen nicht zugänglichen Schaltkontakt (52,51), der nur geschlossen wird von einem an der SLA befindlichen schlüsselähnlichen Teil (45) nach korrekter Befestigung der SLA am Gerät,
sowie je nach Sicherheitsanforderung einem im Inneren des Gerätes im Strahlengang des Lasers befindlichen zusätzlichen mechanischen Verschluß (51), der sich zwingend schließt während der Zeit der Entfernung der SLA vom Laser-Gerät und den Austritt eines Laserstrahles sicher verhindert.

10. Sicherheits-Laserstrahlaustrittsöffnungen nach Anspruch 1, dadurch gekennzeichnet, daß die SLA mindestens eine sicherheitstechnische Vorrichtung der Ansprüche 2 bis 9 enthält oder entsprechend den Anforderungen eine Kombination aus diesen Ansprüchen.

## Claims

1. Safety laser beam apertures (SLAs) for therapeutic and diagnostic medical equipment for the performance levels of soft and mid-range lasers subject to especially high safety requirements,
**characterized in that**
the equipment fitted with the safety laser beam apertures, being inherently safe laser systems which are highly secure against deception and manipulation and which comply with the various safety requirements imposed by different countries, can be used elsewhere than medical treatment rooms and/or autonomously, without constant supervision by trained medical personnel,
and that the SLAs must be covered from the outside, preferably with organic cells or cell aggregates of human or animal skin, in order that, for this time only, the laser supply current be switched on, ie, the laser's activating and operating lock-out devices be switched off, by means of any of the alternative features a) to f) listed below and at the same time the laser beam be prevented from exiting freely into the room, the SLAs having one or more of the following features:
a) a reflected-light ranging sensor (2,3,5,6,7,8) in the direct radiation zone of the laser beam and having a second light source,
and/or
b) a high-frequency (1,15,16,17,18,19,20,21,24,28) or inductive (9,10,11,12,13,14) or capacitive (1,15,16,17,18,19,20,21,24,27,28) proximity switch
and/or
c) at least two sensor contacts (1,22,23,24,25,26,27) which respond to the electrical resistance of the material in contact with them,
and/or
d) at least one sensor contact (1,24,27,28,29) arranged around the SLA at the outside edge thereof, which, as a result of the material of which it is made, responds electrically to the pressure of the material in contact with it,
and/or
e) three sensor contacts (1,32,33,34,35,36,37) connected by means of a pressure plate, which trigger the common switching contact by means of an AND gate (38),
and/or
f) a mechanical closure means for the aperture (1,39,40,41,42,43,44), having an electrical switching contact.

2. The safety laser beam apertures of claim 1,
**characterized in that**
the SLAs have at least one reflected-light ranging sensor whose light beam, emitted from a radiation source (4), is directed - through a beam-limiting channel which may have an anti-reflective coating - from the side, between the laser-beam exit window and the outer limiting edge of the SLA, in such a way onto the axis of the exiting laser beam that it intersects the laser beam immediately in front of the SLA, it being essential that the material to be irradiated by the laser is in this area (8) in order that said material be detected and recognised by a terminating optical element (6) that may be equipped with a filter (7) and is located to the side in a beam-limiting channel 5, which again may have an anti-reflective coating, and, by means of a sensor, serve to switch off the laser device's activating lock-out, with the possibility of simultaneous photometric determination of the material.

3. The safety laser beam apertures of claim 1,
**characterized in that**
the SLAs are equipped on the outside with a proximity switch which surrounds the laser beam aperture and recognises, either inductively (9,10,11,12,13,14), capacitively (1,15,16,17,18,19,20,21,24,27,28) or by means of high-frequency (1,15,16,17,18,19,20,21,24,28), the closely approaching material, and, for the time the material is in close proximity, switches off the laser's activating and operating lock-out devices, with the added possibility of using the high-frequency electromagnetic field therapeutically or to modulate the laser.

4. The safety laser beam apertures of claim 1,
**characterized in that**
the SLAs have at least two outwardly disposed sensor contacts (1,22,23,24,25,26,27) located around the laser beam aperture, which respond to the conductivity of the material touching them and only switch off the laser's activating and operating lock-out devices for as long as said material is touching all the sensor contacts at the same time.

5. The safety laser beam apertures of claim 4,
**characterized in that**
the sensor contacts have a slightly convex outer contact surface (30) and lateral electrical insulation (31).

6. The safety laser beam apertures of claim 1,
**characterized in that**
the SLAs have one or more outwardly disposed sensor contacts (1,24,27,28,29) located around the laser beam aperture, which incorporate(s) semiconductor polymer film or piezoxide materials or strain gauges and which respond(s) directly to the pressure of the material touching them/it and switch(es) off the laser's activating and operating lock-out devices for as long as the material touching them/it exerts the set pressure.

7. The safety laser beam apertures of claim 1,
**characterized in that**
the SLAs contain a mechanical closure means for the laser beam aperture, said inherently safe closure means - in the form of an eccentric disk (43) on a rotatably mounted axle (39) which is perpendicular to but laterally displaced relative to the longitudinal axis of the laser beam - responding to the mechanical pressure of the material covering the beam path, and only unblocking the beam path on being actuated by the material to be irradiated, while at the same time, by means of an electrical contact (44), switching off the laser's activating and operating lock-out devices, and that on removal of the material to be irradiated from the SLA a spiral spring (40) causes the mechanical closure means to close automatically and to switch on the laser's activating lock-out device by means of a switch (44).

8. The safety laser beam apertures according to claims 4-7,
**characterized in that**
the exterior contact surface of the sensors or the mechanical closure means consists of material with germ-inhibiting or germicidal properties, which has good electrical conductivity and which protects against corrosion, while at the same time having low allergenic potential, such as silver, copper, gold, platinum and compounds thereof.

9. The safety laser beam apertures of claim 1,
**characterized in that**
on removal of the SLA together with its sensors and contact surfaces (49) from the laser device (50), the laser's current supply is automatically interrupted by a switching contact (52, 51) which is inaccessible from the outside and which is only closed, once the SLA has been correctly attached to the laser device, by a key-like component (45) attached to the SLA,
and that, depending on the safety requirements, them is an additional mechanical closure means (51) located inside the device in the laser beam path, which is automatically closed for as long as the SLA is removed from the laser device and thus ensures that no laser beam can exit from the device.

10. The safety laser beam apertures of claim 1,
**characterized in that**
the SLAs have at least one of the safety devices claimed in claims 2 to 9, or, depending on the requirements, a combination of these claims.

## Revendications

1. Ouvertures de sécurité pour rayon laser (OSL), destinées aux appareils médicaux de diagnostic et de traitement du domaine de puissance des MID et SOFT lasers devant répondre à des critères de sécurité particulièrement stricts, caractérisées par le fait que les appareils équipés de ces ouvertures de sécurité pour rayon laser sont des systèmes à sécurité intrinsèque, assurant une haute sécurité contre les leurres et une grande sûreté de manipulation, qui autorisent une utilisation à l'extérieur des salles de traitement médical et/ou une utilisation autonome sans surveillance continue par un personnel médical spécialisé, conformément aux dispositions de sécurité, de rigueur variable, en vigueur selon les pays; caractérisées par le fait que ces ouvertures doivent être couvertes de l'extérieur, de préférence par des cellules ou agrégats cellulaires organiques ou par de la peau humaine ou animale, pour que, pendant le temps uniquement où ces ouvertures sont couvertes, l'alimentation électrique du laser soit mise en circuit au moyen des options a) à f) suivantes, ou que le dispositif de verrouillage de la mise en marche ou du fonctionnement du laser soit débloqué et que, parallèlement, la sortie incontrôlée du rayon laser dans la pièce soit évitée; et caractérisées par le fait que ces ouvertures sont dotées de l'une ou de plusieurs des caractéristiques suivantes:
a) un capteur de réflexion lumineuse de proximité (2, 3, 5, 6, 7, 8), assurant la détection au moyen d'une deuxième source de lumière située dans la zone directe de rayonnement du rayon laser et/ou
b) un déclencheur de proximité à haute fréquence (1, 15, 16, 17, 18, 19, 20, 21, 24, 28) ou inductif (9, 10, 11, 12, 13, 14) ou capacitif(1, 15, 16, 17, 18, 19, 20, 21, 24, 27, 28) et/ou
c) au minimum deux contacts de détection (1, 22, 23, 24, 25, 26, 27) qui réagissent à la résistance électrique du matériau en contact avec eux et/ou
d) au minimum un contact de détection placé sur le bord extérieur, autour de l'OSL (1, 24, 27, 28, 29), qui, du fait de son matériau de fabrication, réagit électriquement aux forces de pression exercées par le matériau en contact avec lui et/ou
e) trois contacts de détection (1, 32, 33, 34, 35, 36, 37), reliés par une plaque de pression, qui déclenchent le contact de commutation de commande commun par l'intermédiaire d'un circuit ET (38) et/ou
f) un dispositif de fermeture mécanique de l'ouverture de sortie du rayon laser (1, 39, 40, 41, 42, 43, 44) doté d'un contact de commutation de commande électrique.

2. Ouvertures de sécurité pour rayon laser selon la revendication 1, caractérisées par le fait que ces OSL possèdent au minimum un capteur de réflexion lumineuse de proximité, dont le rayon lumineux émis est, depuis sa source (4) et à travers un canal pouvant être doté d'un revêtement antireflet, limitant le rayonnement et situé latéralement entre la fenêtre de sortie du rayon laser et le bord extérieur de l'OSL, orienté de telle sorte sur l'axe du rayon laser sortant qu'il coupe celui-ci directement devant l'OSL; caractérisées par le fait que le matériau à irradier par le rayon laser doit obligatoirement se trouver dans cette zone (8) pour être détecté par le dernier élément du dispositif optique (6), situé en retrait, latéralement, dans le canal (5), lequel peut lui aussi être doté d'un revêtement antireflet et éventuellement d'un filtre (7), et servir, par l'intermédiaire du capteur, à débloquer le dispositif de verrouillage de l'appareil laser, en offrant parallèlement une possibilité de mesure photométrique du matériau.

3. Ouvertures de sécurité pour rayon laser selon la revendication 1, caractérisées par le fait que ces OSL présentent à l'extérieur, autour de l'ouverture de sortie du rayon laser, un déclencheur de proximité qui détecte, sur le mode inductif (9, 10, 11, 12, 13, 14), capacitif(1, 15, 16, 17, 18, 19, 20, 21, 24, 27, 28) ou à haute fréquence (1, 15, 16, 17, 18, 19, 20, 21, 24, 28), le matériau se rapprochant étroitement de l'ouverture et débloque le dispositif de verrouillage de la mise en marche ou du fonctionnement du laser pendant la durée de cette approche, déclencheur dont les hautes fréquences peuvent être utilisées pour la modulation du laser ou comme vibrations électromagnétiques à usage thérapeutique.

4. Ouvertures de sécurité pour rayon laser selon la revendication 1, caractérisées par le fait que ces OSL comportent au minimum deux contacts de détection (1, 22, 23, 24, 25, 26, 27) placés à l'extérieur, autour des ouvertures de sortie du rayon laser, qui réagissent à la conductivité électrique du matériau en contact avec eux et débloquent le verrouillage de la mise en marche et du fonctionnement du laser uniquement lors du contact simultané de ce matériau avec tous les contacts de détection, pendant la durée de ce contact.

5. Ouvertures de sécurité pour rayon laser selon la revendication 4, caractérisées par le fait que les contacts de détection présentent un léger bombement (30) vers l'extérieur en direction de la surface de contact, et comportent latéralement une isolation électrique (31).

6. Ouvertures de sécurité pour rayon laser selon la revendication 1, caractérisées par le fait que ces OSL comportent à l'extérieur, autour des ouvertures de sortie du rayon laser, un ou plusieurs contacts de détection (1, 24, 27, 28, 29), fabriqué(s) à partir de feuilles polymères semi-conductrices, de matériaux piézoxydes ou de jauges de contrainte, qui réagit/réagissent directement aux forces de pression qu'exerce le matériau en contact avec lui/eux et qui débloque/débloquent le verrouillage de la mise en marche ou du fonctionnement du laser pour la pression donnée, pendant le temps où cette pression est exercée par le matériau en contact avec lui/eux.

7. Ouvertures de sécurité pour rayon laser selon la revendication 1, caractérisées par le fait que ces OSL comportent un système mécanique de fermeture de l'ouverture de sortie du rayon laser qui se déclenche sous l'effet de la pression mécanique exercée par le matériau interrompant la marche du rayon laser, système à sécurité intrinsèque de type excentrique (43) à axe rotatif (39), positionné en direction transversale par rapport à l'axe du rayon laser, qui libère la marche du rayon laser uniquement lorsqu'il est actionné par le matériau à irradier et débloque parallèlement le verrouillage de la mise en marche ou du fonctionnement du laser par l'intermédiaire d'un contact électrique (44), système qui, lorsqu'on éloigne le matériau à irradier de l'OSL, se ferme automatiquement par l'intermédiaire d'un ressort spiral (40), et enclenche le dispositif de verrouillage de la mise en marche du laser par le biais d'un interrupteur (44).

8. Ouvertures de sécurité pour rayon laser selon les revendications 4 + 5 + 6 + 7, caractérisées par le fait que la surface de contact extérieure des capteurs ou du dispositif mécanique de fermeture est constitué d'un matériau inhibiteur de la germination ou germicide, possédant une bonne conductivité électrique ainsi que des propriétés anti-corrosion, tout en présentant un potentiel allergène réduit, comme l'argent, le cuivre, l'or, le platine et leurs composés.

9. Ouvertures de sécurité pour rayon laser selon la revendication 1, caractérisées par le fait qu'en cas de retrait de l'OSL, y compris des surfaces des capteurs et des contacts (49), de l'appareil laser (50), l'alimentation en courant du laser est obligatoirement interrompue par le biais d'un contact de commutation de commande (52, 51) non accessible de l'extérieur qui se ferme uniquement par l'intermédiaire d'un élément similaire à une clé (45) situé sur l'OSL, pour autant que l'OSL soit correctement fixée sur l'appareil, ainsi que, selon les exigences de sécurité requises, par l'intermédiaire d'un dispositif mécanique de fermeture supplémentaire (51) se trouvant à l'intérieur de l'appareil, sur le trajet du rayon laser, dispositif qui se ferme obligatoirement pendant la durée de retrait de l'OSL de l'appareil laser et empêche avec fiabilité toute sortie d'un rayon laser.

10. Ouvertures de sécurité pour rayon laser selon la revendication 1, caractérisées par le fait que ces OSL comportent au minimum un des dispositifs techniques de sécurité selon les revendications 2 à 9, ou une combinaison de ces revendications, en fonction des exigences requises.
